# EUROPEAN PATENT APPLICATION

(11) **EP 4 389 130 A1**
(43) Date of publication of application: **26.06.2024**
(21) Application number: 22857846.4
(22) Date of filing: 17.08.2022
(51) Int. Cl.: A61K 31/575, A61P 27/12

(54) **APPLICATION OF STEROID COMPOUND IN PREPARATION OF DRUG FOR PREVENTING AND/OR TREATING EYE FLOATERS**

(30) Priority: 18.08.2021 CN 202110951369
(71) Applicant: Guangzhou Ocusun Ophthalmic Biotechnology Co., Ltd., Guangzhou, Guangdong 511400 (CN); Ocusun Ophthalmic Pharmaceutical (Guangzhou) Co., Ltd., Guangzhou, Guangdong 511400 (CN)
(72) Inventor: WANG, Yandong, Guangzhou, Guangdong 511400 (CN); SU, Yingxue, Guangzhou, Guangdong 511400 (CN); CAO, Chen, Guangzhou, Guangdong 511400 (CN); WU, Meirong, Guangzhou, Guangdong 511400 (CN); XUE, Yaping, Guangzhou, Guangdong 511400 (CN); YU, Chuiliang, Guangzhou, Guangdong 511400 (CN)
(74) Representative: Latscha Schöllhorn Partner AG
(86) International application number: PCT/CN2022/113024
(87) International publication number: WO 2023/020535

(57) **Abstract**

An application of a steroid compound in the preparation of a drug for preventing and/or treating eye floaters. The steroid compound can have good therapeutic, alleviative, and preventive effects on eye floaters, can alleviate and/or cure eye floaters to a great extent, and can improve visual clarity.

## Description

### TECHNICAL FIELD

The present invention belongs to the field of chemical medical technology, and relates to an application of a steroid compound in the preparation of a medicament for preventing and/or treating of floaters.

### BACKGROUND

Muscae volitantes (Floaters) are natural aging phenomena, generally caused by vitreous degeneration. As one ages, the vitreous "liquefy" and produce some turbid substances. Therefore, the official name of floaters is "vitreous opacity" or "vitreous floats". Moving black specks are saw in front of the eyes, like flying mosquitoes, hence the name. Floaters are caused by opaque objects in the vitreous projection on the retina, which are more noticeable against bright light or a white background, and can even be depicted in different shapes by sensitive people. Many floaters persist for a long time, remain unchanged all year round, do not affect vision, and there are no eye organ lesions after examination, often do not require treatment, and there is no effective treatment.

Some elderly people suddenly have one or two dark shadows in front of their eyes and are not accompanied by other symptoms, which is often due to detachment of the posterior vitreous membrane, and generally not very harmful. However, if a large number of black spots appear suddenly, retinal vascular rupture or hemorrhage or retinal tear formation should be taken into account, which may be a precursor to retinal detachment, and the fundus should be examined in further detail. Many inflammatory cells or exudates can enter the vitreous during choroiditis, which is a common cause of pathological floaters, and often difficult to be detected because of the presence of visual impairment. The floaters felt by myopia patients are often related to the colliquation of the vitreous.

Floaters are very special and common eye diseases. A few of floaters can seriously threaten eyeballs, while most floaters are benign, or "physiologically floaters", and generally do not require treatment. But even so, floaters can seriously affect the physical and mental health of patients. Although some drugs on the market can alleviate symptoms, but they have relatively large side effects. At present, there is no drug that has a definite curative effect on this disease. Therefore, researching a drug for the treatment of floaters with good clinical effect and few side effects is an urgent problem to be solved.

### SUMMARY OF THE PRESENT INVENTION

In view of the deficiencies in the prior art, an object of the present invention is to provide a steroid compound in the preparation of a medicament for preventing and/or treating floaters. The steroidal compound according to the present invention can have good therapeutic, alleviative, and preventive effect on floaters, can alleviate and/or cure floaters to a great extent, and can improve visual clarity.

For achieving this purpose, the present invention adopts the following technical solutions.

In the first aspect, the present invention provides a use of a steroidal compound in the preparation of a medicament for preventing and/or treating floaters, wherein the steroidal compound has a structure shown in formula I, or is a stereoisomer, tautomer, nitrogen oxide, solvate, metabolite, pharmacologically acceptable salt or prodrug of the compound of the structure shown in formula I; 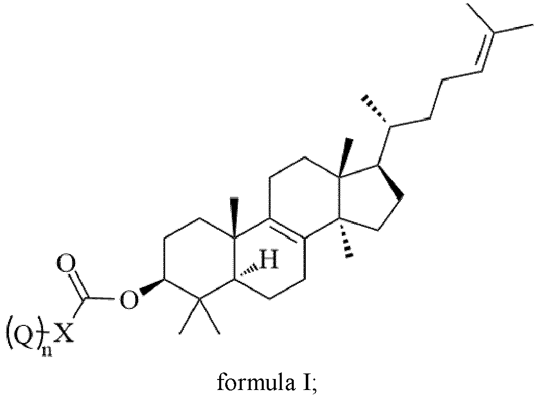
wherein, X is selected from the group consisting of aryl, heteroaryl, cycloalkyl, heterocycloalkyl, heteroalkyl, and substituted or unsubstituted alkyl, and the substituted group is selected from the group consisting of hydroxyl, sulfhydryl, amino, aryl, heteroaryl, carboxyl, R¹R²NC(=O)- and R¹R²NC(=NH)-NR³-;
R¹ and R² contained in R¹R²NC(=O)- or R¹R²NC(=NH)-NR³- in the present invention are both connected to N.
Each Q is independently selected from the group consisting of -H, -D, halogen, hydroxyl, amino, cyano, nitro, carboxyl, alkyl carbonyl, alkyl, haloalkyl, cycloalkyl, heterocyclyl, aryl or heteroaryl;
the alkyl carbonyl in the present invention refers to R-C(=O)-, in which R is alkyl, and the carbonyl carbon is connected to X.
n is 0, 1, 2, or 3.
R¹, R², and R³ are independently selected from the group consisting of -H, -D, and alkyl.
As a preferred technical solution of the present invention, X is selected from the group consisting of C₆₋₁₀ aryl, C₂₋₉ heteroaryl, C₃₋₈ cycloalkyl, C₂₋₁₀ heterocycloalkyl, C₁₋₆ heteroalkyl, and substituted or unsubstituted C₁₋₆ alkyl, and if the C₁₋₆ alkyl has a substituent group, the substituent group is selected from the group consisting of hydroxyl, sulfhydryl, amino, C₆₋₁₀ aryl, C₂₋₉ heteroaryl, carboxyl, R¹R²NC(=O)- and R¹R²NC(=NH)-NR³-; and
R¹, R², and R³ are independently selected from the group consisting of -H, -D, and C₁₋₆ alkyl.

The C₆₋₁₀ can be C6, C7, C8, C9, or C10. The C₂₋₉ can be C3, C4, C5, C6, C7, or C8. The C₃₋₈ can be C4, C5, C6, or C7. The C₂₋₁₀ can be C3, C4, C5, C6, C7, C8, or C9. The C₁₋₆ can be C2, C3, C4, or C5.

As a preferred technical solution of the present invention, X is selected from the group consisting of methyl, ethyl, propyl, isopropyl, n-butyl, isobutyl, tert-butyl, hydroxymethyl, hydroxyethyl, mercaptomethyl, mercaptoethyl, aminomethyl, aminoethyl, aminopropyl, phenyl methyl, phenyl ethyl, imidazolyl methyl, carboxymethyl, carboxyethyl, methylthiomethyl, methylthioethyl, phenyl, naphthyl, cyclopropyl, cyclobutyl, cyclopentyl, cyclohexyl, tetrahydrofuranyl, dihydrofuranyl, tetrahydrothiophenyl, dihydrothiophenyl, 1,3-dioxycyclopentyl, dithiocyclopentyl, tetrahydropyranyl, dihydropyranyl, 2H-pyranyl, 4H-pyranyl, tetrahydrothianyl, piperidinyl, morpholinyl, thiomorpholinyl, piperazinyl, dioxanyl, dithianyl, thioxanyl, homopiperazinyl, homopiperidinyl, oxacycloheptyl, thiacycloheptyl, oxazanyl, diazanyl, thiazanyl, indolinyl, 1,2,3,4-tetrahydroquinolinyl, 1,2,3,4-tetrahydroisoquinolinyl, furanyl, imidazolyl, 3-isooxazolyl, isooxazolyl, oxazolyl, pyrrolyl, pyridinyl, pyrimidinyl, pyridazinyl, thiazolyl, tetrazolyl, triazolyl, 2-thiophenyl, 3-thiophenyl, pyrazolyl, isothiazolyl, 1,2,3-oxadiazolyl, 1,2,5-oxadiazolyl, 1,2,4-oxadiazolyl, 1,2,3-triazolyl, 1,2,3-thiodiazolyl, 1,3,4-thiodiazolyl, 1,2,5-thiodiazolyl, pyrazinyl, 1,3,5-triazinyl, benzimidazolyl, benzofuranyl, benzothiophenyl, indolyl, purinyl, quinolinyl, isoquinolinyl, imidazolo[1,2-a]pyridinyl, pyrazolo[1,5-a]pyridinyl, pyrazolo[1,5-a]pyrimidinyl, imidazolo[1,2-b]pyridazinyl, [1,2,4]triazolo[4,3-b]pyridazinyl, [1,2,4]triazolo[1,5-a]pyrimidinyl, [1,2,4]triazolo[1,5-a]pyridinyl, 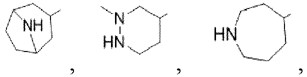 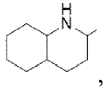 C₁₋₃ alkyl with H₂NC(=O)-substituent or C₁₋₃ alkyl with H₂NC(=NH)-NH-substituent.

As a preferred technical solution of the present invention, each Q is independently selected from the group consisting of -H, -D, halogen, hydroxyl, amino, cyano, nitro, carboxyl, C₁₋₆ alkyl carbonyl, C₁₋₆ alkyl, C₁₋₆ haloalkyl, C₃₋₈ cycloalkyl, C₂₋₉ heterocyclyl, C₆₋₁₀ aryl and C₂₋₉ heteroaryl.

The C₁₋₆ can be C2, C3, C4, or C5. The C₃₋₈ can be C4, C5, C6, or C7. The C₂₋₉ can be C3, C4, C5, C6, C7, or C8. The C₆₋₁₀ can be C6, C7, C8, C9, or C10.

As a preferred technical solution of the present invention, each Q is independently selected from the group consisting of -H, -D, -F, -Cl, -Br, hydroxyl, amino, cyano, carboxyl, formyl, acetyl, methyl, ethyl, propyl, isopropyl, n-butyl, isobutyl, tert-butyl, trifluoromethyl, difluoromethyl, phenyl, naphthyl, cyclopropyl, cyclobutyl, cyclopentyl, cyclohexyl, tetrahydrofuranyl, dihydrofuranyl, tetrahydrothiophenyl, dihydrothiophenyl, 1,3-dioxycyclopentyl, dithiocyclopentyl, tetrahydropyranyl, dihydropyranyl, 2H-pyranyl, 4H-pyranyl, tetrahydrothianyl, piperidinyl, morpholinyl, thiomorpholinyl, piperazinyl, dioxanyl, dithianyl, thioxanyl, homopiperazinyl, homopiperidinyl, oxacycloheptyl, thiacycloheptyl, oxazanyl, diazanyl, thiazanyl, indolinyl, 1,2,3,4-tetrahydroquinolinyl, 1,2,3,4-tetrahydroisoquinolinyl, furanyl, imidazolyl, 3-isooxazolyl, isooxazolyl, oxazolyl, pyrrolyl, pyridinyl, pyrimidinyl, pyridazinyl, thiazolyl, tetrazolyl, triazolyl, 2-thiophenyl, 3-thiophenyl, pyrazolyl, isothiazolyl, 1,2,3-oxadiazolyl, 1,2,5-oxadiazolyl, 1,2,4-oxadiazolyl, 1,2,3-triazolyl, 1,2,3-thiodiazolyl, 1,3,4-thiodiazolyl, 1,2,5-thiodiazolyl, pyrazinyl, 1,3,5-triazinyl, benzimidazolyl, benzofuranyl, benzothiophenyl, indolyl, purinyl, quinolinyl, isoquinolinyl, imidazolo[1,2-a]pyridinyl, pyrazolo[1,5-a]pyridinyl, pyrazolo[1,5-a]pyrimidinyl, imidazolo[1,2-b]pyridazinyl, [1,2,4]triazolo[4,3-b]pyridazinyl, [1,2,4]triazolo[1,5-a]pyrimidinyl, and [1,2,4]triazolo[1,5-a]pyridinyl.

As a specific embodiment of the present invention, the compound of the structure shown in formula I is selected from the group consisting of any one of the following compounds:

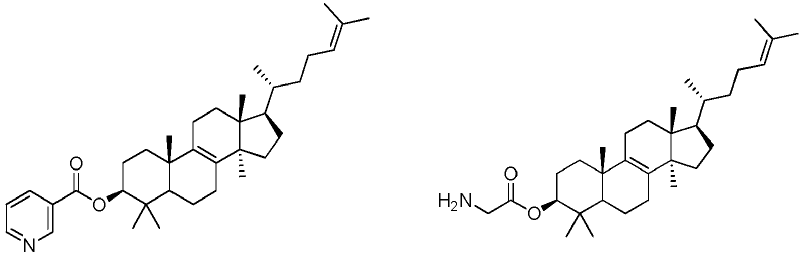

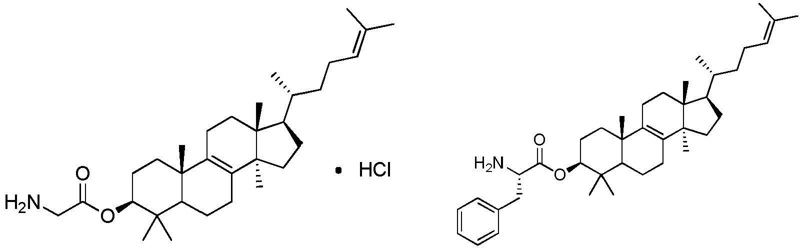

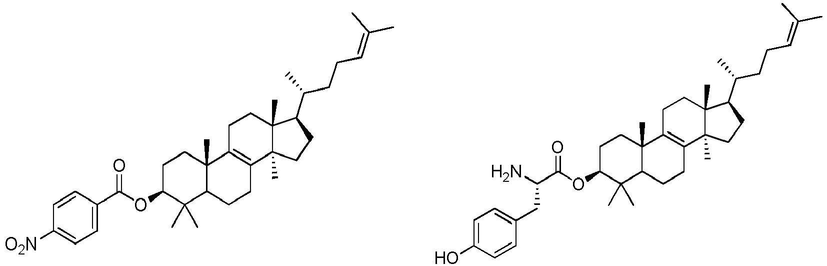

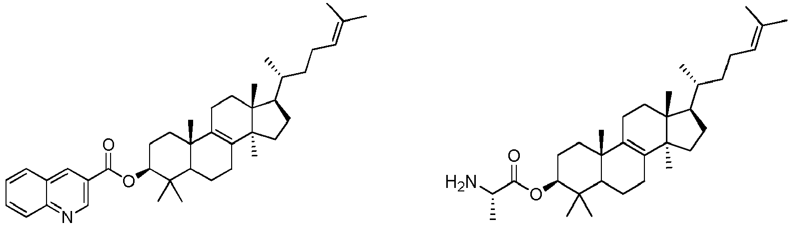

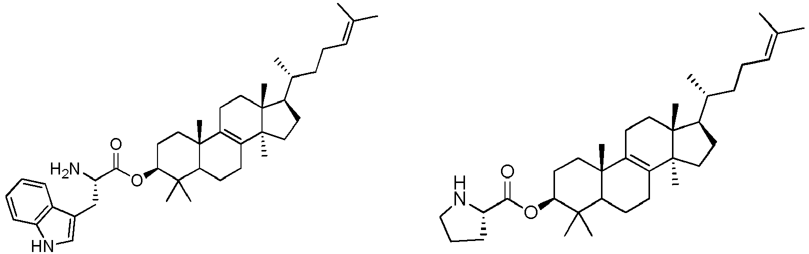

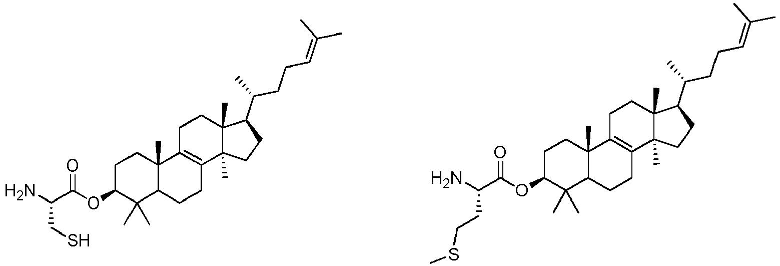

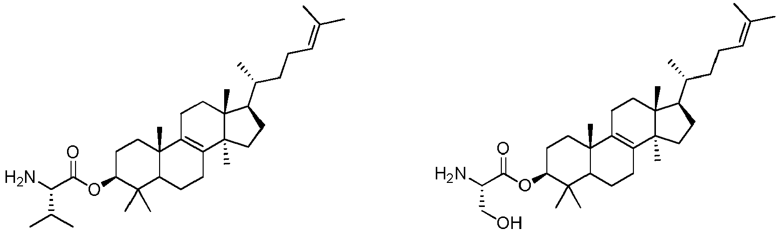

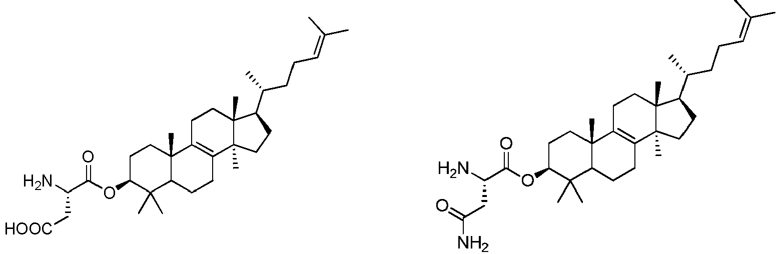

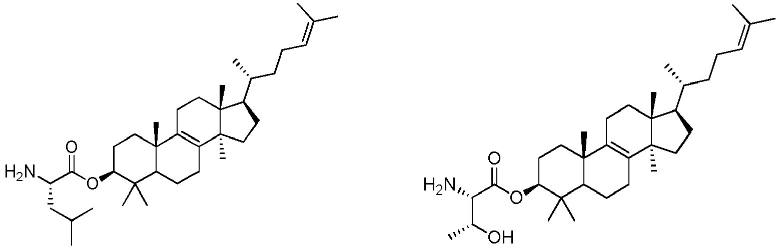

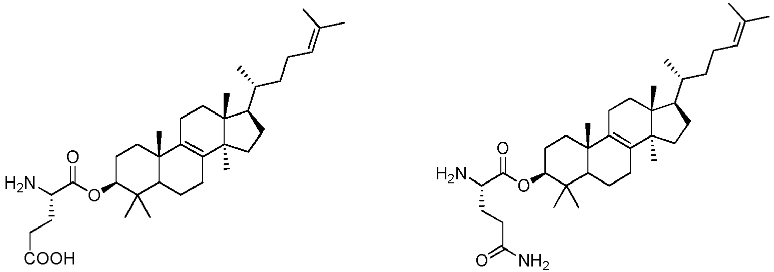

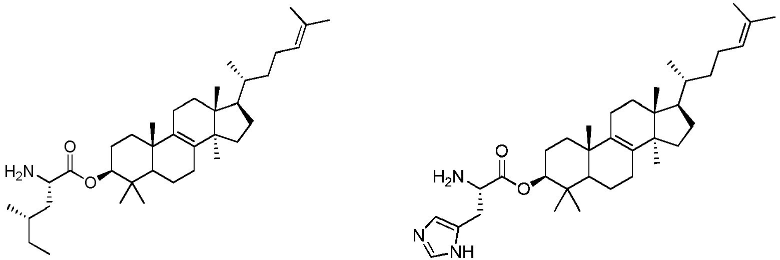

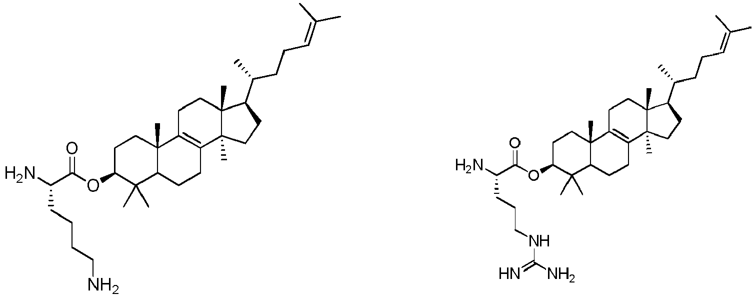

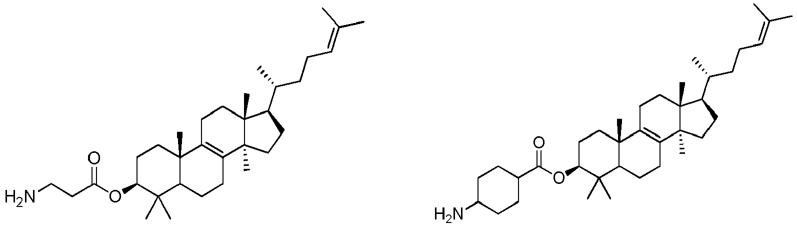

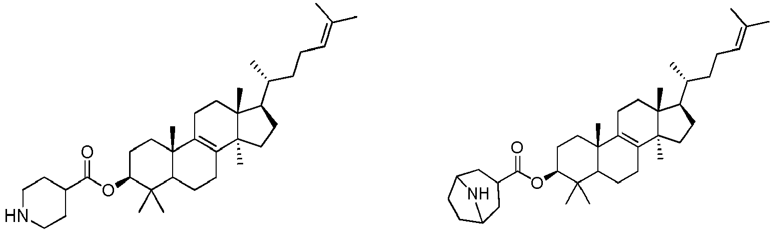

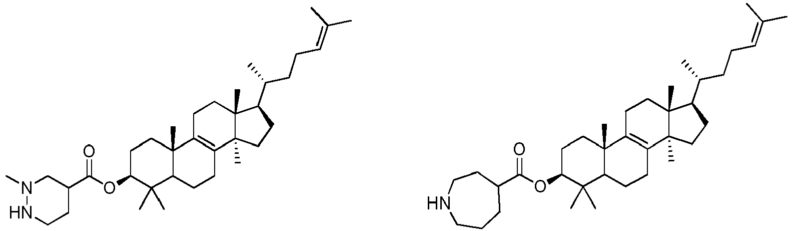

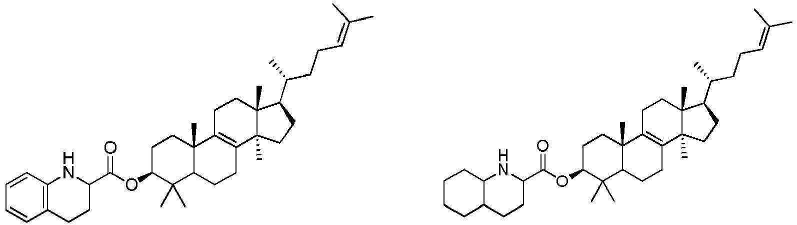

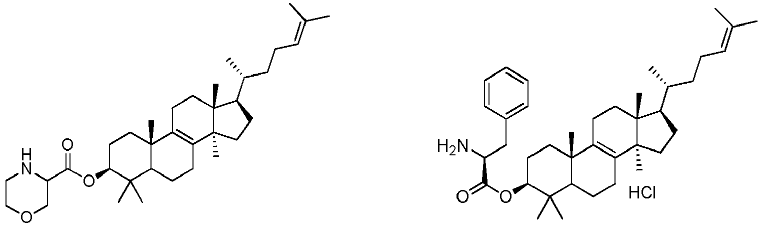

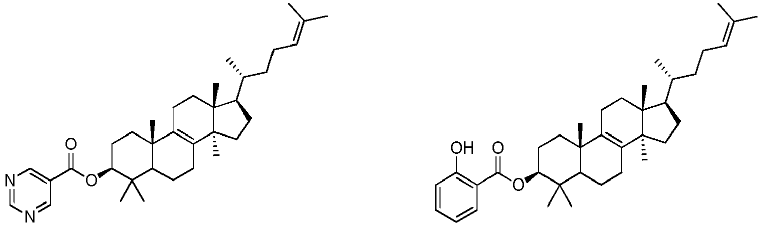

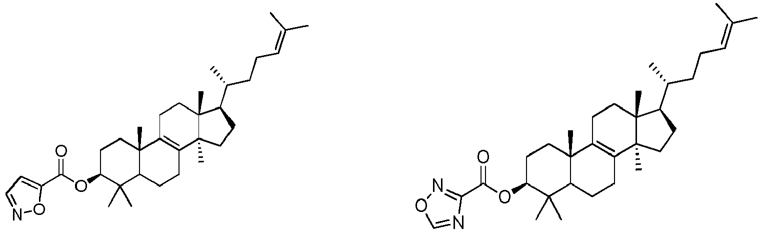

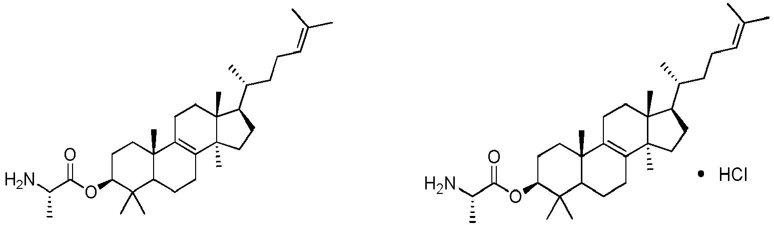

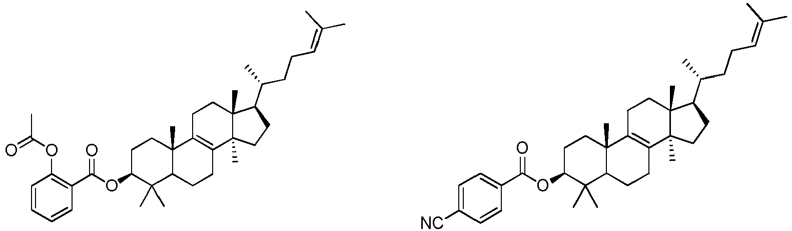

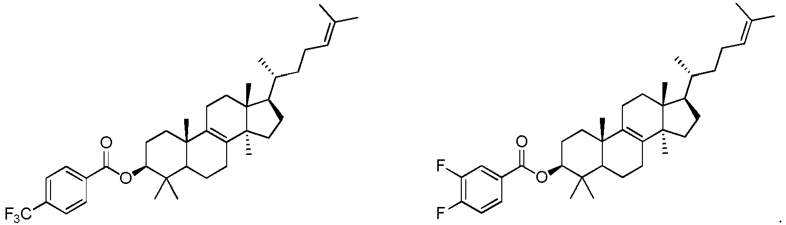

The steroidal compound provided by the present invention may be used as a unprocessed chemical drug for treatment or as an active ingredient in a pharmaceutical composition.

In the second aspect, the present invention provides a use of a composition in the preparation of a medicament for preventing and/or treating floaters, wherein the composition comprises the steroidal compound of the first aspect, and one or more of the pharmaceutically acceptable carriers, excipients, diluents, adjuvants or vehicles.

Substances that may be used as pharmaceutically acceptable carriers include, but not limited to, ion exchangers; aluminum; aluminum stearate; lecithin; serum proteins, such as human serum protein; buffering substances such as phosphate; glycine; sorbic acid; potassium sorbate; partial glyceride mixtures of saturated vegetable fatty acids; water; salts; electrolytes, such as protamine sulfate; disodium hydrogen phosphate; potassium hydrogen phosphate; sodium chloride; zinc salts; colloidal silicon; magnesium trisilicate; polyvinylpyrrolidone; polyacrylate; waxes; polyethylene-polyoxypropylene-blocking polymers; lanolin; sugars, such as lactose, glucose and sucrose; starches such as corn starch and potato starch; cellulose and derivatives thereof such as sodium carboxymethylcellulose, ethylcellulose and cellulose acetate; gum powder; malt; gelatin; talcum powder; excipients such as cocoa bean butter and suppository wax; oils such as peanut oil, cottonseed oil, safflower oil, sesame oil, olive oil, corn oil and soybean oil; diols compounds, such as propylene glycol and polyethylene glycol; esters such as ethyl oleate and ethyl laurate; agar; buffers such as magnesium hydroxide and aluminum hydroxide; alginic acid; pyrogen free water; isotonic salt; Ringer's solution; ethanol; phosphate buffered solutions; and other non-toxic suitable lubricants such as sodium laurylsulfate and magnesium stearate, colorants, releasers, coating materials, sweeteners, flavorings and fragrances, preservatives and antioxidants.

Compared with the prior art, the present invention has the following beneficial effects.

It is found in the present invention that the steroidal compound provided by the present invention can be used to prevent, dispose, treat or alleviate floaters in patients, and the side effects are minimal or basically free.

### DETAILED DESCRIPTION OF THE EMBODIMENT

The technical solution of the present invention is further described below by specific embodiments. Those skilled in the art should be clear that the specific embodiments are merely to aid in understanding the present invention and should not be regarded as a specific limitation of the present invention.

Unless otherwise indicated, all scientific and technical terms used in the present invention have the same meanings as commonly understood by those skilled in the art to which the present invention belongs.

The term "stereoisomer" described in the present invention refers to a compound with the same chemical structure, but the atoms or groups are arranged in different ways. Stereoisomers include enantiomers, diastereomers, conformational isomers (rotational isomers), geometric isomers (cis/trans) isomers, rotational resistance isomers, and so on.

Depending on the choice of starting materials and method, the compounds of the present invention may exist in the form of one of the possible isomers or a mixture of them, such as mixtures of racemates and enantiomers (which depend on the number of asymmetric carbon atoms). Optically active (R)- or (S)-isomers can be prepared using chiral synthons or chiral reagents, or resolved using conventional techniques. If the compound contains a double bond, the substituent may be in E or Z configuration, and if the compound contains a disubstituted cycloalkyl, the substituent in the cycloalkyl may be in cis or trans configuration.

The resulting mixture of any stereoisomers can be separated into pure or essentially pure geometric isomers, enantiomers, diastereomers, e.g., by chromatography and/or fractional crystallization, depending on the differences in the physicochemical properties of the components.

Unless otherwise indicated, the structural formula described in the present invention includes all isomeric forms (such as enantiomers, diastereomers, and geometric isomers (or conformational isomers): e.g., R and S configurations containing asymmetric centers, (Z) and (E) isomers of double bonds, and conformational isomers of (Z) and (E). Therefore, a single stereochemical isomer of the compound in the present invention or mixtures of enantiomers, diastereomers, or geometric isomers (or conformational isomers) thereof are within the scope of the present invention.

The term term "prodrug" used in the present invention represents a compound which can convert into the compound shown in formula I *in vivo.* Such conversion is affected by the hydrolysis of the prodrug in the blood or the enzymatic conversion of the prodrug to the parent structure in the blood or tissues. The prodrug compound of the present invention can be an ester, and in the existing invention the esters that can be used as a prodrug include phenyl esters, aliphatic (C₁₋₂₄) esters, acyloxymethyl esters, carbonates, carbamates and amino acid esters. For example, a compound in the present invention contains a hydroxyl group, that is, the compound can be acylated to form a prodrug. Other forms of prodrugs include phosphate esters, such as those obtained by phosphorylation of the parent hydroxyl group. A complete discussion of prodrugs can be found in the following literature: T. Higuchi and V. Stella, Pro-drugs as Novel Delivery Systems, Vol. 14 of the A.C.S. Symposium Series, Edward B. Roche, ed., Bioreversible Carriers in Drug Design, American Pharmaceutical Association and Pergamon Press, 1987, J. Rautio et al, Prodrugs: Design and Clinical Applications, Nature Review Drug Discovery, 2008, 7, 255-270, and S. J. Hecker et al, Prodrugs of Phosphates and Phosphonates, Journal of Medicinal Chemistry, 2008, 51, 2328-2345.

The racemates of any resulting final product or intermediate may be separated into optical enantiomers by a method familiar to those skilled in the art, e.g., by separating the obtained salts of its diastereomer. Racemic products can also be separated by chiral chromatography, e.g., high-performance liquid chromatography (HPLC) using chiral adsorbents. In particular, enantiomers can be prepared by asymmetric synthesis, e.g., see Jacques, et al., Enantiomers, Racemates and Resolutions (Wiley Interscience, New York, 1981); Principles of Asymmetric Synthesis (2nd Ed. Robert E. Gawley, Jeffrey Aubé, Elsevier, Oxford, UK, 2012); Eliel, E.L. Stereochemistry of Carbon Compounds (McGraw-Hill, NY, 1962); Wilen, S.H. Tables of Resolving Agents and Optical Resolutions p. 268 (E.L. Eliel, Ed., Univ. of Notre Dame Press, Notre Dame, IN 1972); Chiral Separation Techniques: A Practical Approach (Subramanian, G. Ed., Wiley-VCH Verlag GmbH & Co. KGaA, Weinheim, Germany, 2007).

The term "tautomer" or "tautomeric form" refers to structural isomers with different energies that can be converted into each other through a low energy barrier. If tautomerism is possible (e.g. in solution), the chemical equilibrium of the tautomer can be achieved. For example, a proton tautomer (also known as a prototropic tautomer) includes interconversion through proton migration, such as keto-enol isomerization and imine-enamine isomerization. A valence tautomer includes interconversion through the recombination of some bond-forming electrons. A specific example of keto-enol tautomerism are the interconversion between two tautomers of pentane-2,4-dione and 4-hydroxypentyl-3-en-2-one. Another example of tautomerism is phenol-ketone tautomerism. A specific example of phenol-ketone tautomerism is the interconversion between two tautomers of pyridine-4-ol and pyridine-4(1H)-ketone. Unless otherwise indicated, all tautomeric forms of the compounds of the present invention are within the scope of the present invention.

The salt mentioned in the present invention is a pharmaceutically acceptable salt, wherein the term "pharmaceutically acceptable salt" is well known in the art, as recorded in the literature: Berge et al., describe pharmaceutically acceptable salts in detail in J. Pharmacol Sci, 1997, 66, 1-19. Non-limiting examples of pharmaceutically acceptable salts include inorganic salts formed by reaction with an amino group, such as hydrochloride, hydrobromide, phosphate, metaphosphate, sulfate, sulfite, nitrate, perchlorate, and organic salts, such as carboxylate, sulfonate, sulfinate, thiocarboxylate, etc., specifically such as, but not limited to, methanesulfonate, ethanesulfonate, formate, acetate, succinate, benzoate, succinate, dihydroxynaphthoate, salicylate, galactodiate, glucoseheptylate, mandelate, 1,2-ethanedisulfonate, 2-naphthalenesulfonate, carbonate, trifluoroacetate, hydroxyacetate, isethionate, oxalate, maleate, tartrate, citrate, succinate, malonate, benzenesulfonate, p-toluenesulfonate, malate, fumarate, lactate, lactobionate, or oxalic acid, or those salts obtained by other methods recorded in the literature such as ion exchange method. Other pharmacologically acceptable salts include adipate, alginate, ascorbate, aspartate, benzenesulfonate, bisulfate, borate, butyrate, camphorate, camphor sulfonate, cyclopentylpropionate, digluconate, dodecyl sulfate, ethanesulfonate, glucoheptanate, glycerophosphate, gluconate, hemisulfate, enanthate, caproate, hydroiodate, 2-hydroxy-ethanesulfonate, lacturonate, laurate, lauryl sulfate, nicotinate, nitrate, oleate, palmitate, pamoate, pectate, persulfate, 3-phenylpropionate, picrate, valerate, propionate, stearate, thiocyanate, undecanoate, valerate, etc. In addition, pharmaceutically acceptable salts include salts obtained from appropriate bases, such as salts of alkali metals, alkaline earth metals, ammonium, and N⁺(C₁₋₄ alkyl)₄. The present invention also intends to conceive a quaternary ammonium salt formed by any compound containing a group of N. Water-soluble or oil-soluble or dispersed products can be obtained by quaternization. Alkali metals or alkaline earth salts include sodium, lithium, potassium, calcium, magnesium, etc. Pharmaceutically acceptable salts further include appropriate, non-toxic ammonium, quaternary ammonium salts, and amine cations that resist the formation of equilibrium ions, such as halides, carboxylates, sulfates, phosphates, nitrates, C₁₋₈ sulfonates and aromatic sulfonates.

The pharmaceutically acceptable salts can be formed with inorganic and organic acids such as acetate, aspartate, benzoate, benzenesulfonate, bromide/hydrobromide, bicarbonate/carbonate, bisulfate/sulfate, camphor sulfonate, chloride/hydrochloride, chlorophylline salt, citrate, ethanedisulfonate, fumarate, glucoheptanate, gluconate, glucuronate, hippurate, hydroiodate/iodide, isethionate, lactate, lacturonate, lauryl sulfate, malate, maleate, malonate, mandelate, methanesulfonate, methyl sulfate, naphthoate, naphthalenesulfonate, nicotinate, nitrate, octadecate, oleate, oxalate, palmitate, pamoate, phosphate/biphosphate/dihydrogen phosphate, polygalactoate, propionate, stearate, succinate, sulfosalicylate, tartrate, toluenesulfonate, and trifluoroacetate.

Inorganic acids from which salts can be derived include e.g. hydrochloric acid, hydrobromic acid, sulfuric acid, nitric acid, phosphoric acid, etc.

Organic acids from which salts can be derived include such as acetic acid, propionic acid, glycolic acid, oxalic acid, maleic acid, malonic acid, succinic acid, fumaric acid, tartaric acid, citric acid, benzoic acid, mandelic acid, methanesulfonic acid, ethanesulfonic acid, p-toluenesulfonic acid, sulfosalicylic acid, etc.

The term" solvate" of the present invention refers to an associated complex formed by one or more solvent molecules with a compound of the present invention. Solvents that form solvates include, but not limited to, water, isopropanol, ethanol, methanol, dimethyl sulfoxide, ethyl acetate, acetic acid, aminoethanol. The term "hydrate" refers to an associated complex formed by water as a solvent molecule.

The term "pharmaceutical composition" means a mixture of one or more of the compounds according to the present invention, salts or physiologically/pharmacologically acceptable salts or prodrugs thereof, and other chemical components, such as physiologically/pharmacologically acceptable carriers or excipients. The purpose of the pharmaceutical composition is to facilitate the administration of the compound to the organism.

As used in the present invention the term "treatment" for any disease or condition, in some embodiments of the present invention means to improve the disease or condition (i.e., to alleviate or prevent or mitigate the development of the disease or at least one clinical symptom thereof). In other embodiments, "treatment" refers to moderating or improving at least one physical parameter, including physical parameters that may not be perceived by the patient. In other embodiments, "treatment" refers to modulating a disease or condition, either physically (e.g., stabilizing a perceptible symptom) or physiologically (e.g., stabilizing a physical parameter) or both. In other embodiments, "treatment" means to preventing or delaying the onset, occurrence or exacerbation of a disease or condition.

The term "alkyl" used in the present invention means a monovalent hydrocarbon group of 1-20 carbon atoms, or 1-10 carbon atoms, or 1-8 carbon atoms, or 1-6 carbon atoms, or 1-4 carbon atoms, or 1-3 carbon atoms in a saturated linear chain or branched chain, wherein the alkyl can be independently and optionally substituted by one or more substituents described in the present invention. Examples of alkyl include, but not limited to, methyl (Me, -CH₃), ethyl (Et, -CH₂CH₃), n-propyl (n-Pr, -CH₂CH₂CH₃), isopropyl (i-Pr, -CH(CH₃)₂), n-butyl (n-Bu, -CH₂CH₂CH₂CH₃), isobutyl (i-Bu, -CH₂CH(CH₃)₂), sec-butyl (s-Bu, -CH(CH₃)CH₂CH₃), tert-butyl (t-Bu, -C(CH₃)₃), n-pentyl (-CH₂CH₂CH₂CH₂CH₃), 2-pentyl (-CH(CH₃)CH₂CH₂CH₃), 3-pentyl (-CH(CH₂CH₃)₂), 2-methyl-2-butyl (-C(CH₃)₂CH₂CH₃), 3-methyl-2-butyl (-CH(CH₃)CH(CH₃)₂), 3-methyl-1-butyl (-CH₂CH₂CH(CH₃)₂), 2-methyl-1-butyl (-CH₂CH(CH₃)CH₂CH₃), n-hexyl (-CH₂CH₂CH₂CH₂CH₂CH₃), 2-hexyl (-CH(CH₃)CH₂CH₂CH₂CH₃), 3-hexyl (-CH(CH₂CH₃)(CH₂CH₂CH₃)), 2-methyl-2-pentyl (-C(CH₃)₂CH₂CH₂CH₃), 3-methyl-2-pentyl (-CH(CH₃)CH(CH₃)CH₂CH₃), 4-methyl-2-pentyl (-CH(CH₃)CH₂CH(CH₃)₂), 3-methyl-3-pentyl (-C(CH₃)(CH₂CH₃)₂), 2-methyl-3-pentyl (-CH(CH₂CH₃)CH(CH₃)₂), 2,3-dimethyl-2-butyl (-C(CH₃)₂CH(CH₃)₂), 3,3-dimethyl-2-butyl (-CH(CH₃)C(CH₃)₃), n-heptyl, n-octyl, etc. The term "alkyl" and its prefix "alk" used herein both contain saturated carbon chains of linear and branched chains. The term "alkylene" used herein refers to a saturated divalent hydrocarbon group obtained by the elimination of two hydrogen atoms from a linear or branched saturated hydrocarbon, examples of which include, but not limited to, methylene, ethylidyne, sub-isopropyl, etc.

The term "cycloalkyl" refers to a monovalent or polyvalent, non-aromatic, saturated or partially unsaturated ring that does not contain heteroatoms, including a single cyclic ring having 3-12 carbon atoms or a bicyclic ring having 7-12 carbon atoms. A bicyclic carbon ring with 7-12 atoms can be a bicyclic [4,5], [5,5], [5,6] or [6,6] system, while a bicyclic carbon ring with 9 or 10 atoms can be a bicyclic [5,6] or [6,6] system. Suitable cyclic aliphatic groups include, but not limited to, cycloalkyl, cycloalkenyl, and cycloalkynyl. Examples of cyclic aliphatic groups include, but not limited to, cyclopropyl, cyclobutyl, cyclopentyl, 1-cyclopentyl-1-enyl, 1-cyclopentyl-2-enyl, 1-cyclopentyl-3-enyl, cyclohexyl, 1-cyclohexyl-1-enyl, 1-cyclohexyl-2-enyl, 1-cyclohexyl-3-enyl, cyclohexadienyl, cycloheptyl, cyclooctyl, cyclononyl, cyclodecyl, cycloundecyl, cyclododecyl, etc. The "cyclic aliphatic group" or "carbon ring", "carbon ring group", "cycloalkyl" may be substituted or unsubstituted, wherein the substitutes may be, but not limited to, hydroxyl, amino, halogen, cyano, aryl, heteroaryl, alkoxy, alkamino, alkyl, alkenyl, alkynyl, cycloalkyl, heterocyclyl, sulfhydryl, nitro, aryloxy, hydroxyl-substituted alkoxy, hydroxyl-substituted alkyl-C(=O), alkyl-C(=O), alkyl-S(=O), alkyl-S(=O)₂-, hydroxyl-substituted alkyl-S(=O), hydroxyl-substituted alkyl-S(=O)₂, carboxylalkoxy, etc.

The terms "heterocycle", "heterocyclyl", "heteralicyclic group", or "heterocyclic" are used interchangeably herein, and all refer to a single cyclic, bicyclic, or tricyclic system in which one or more carbon atoms on the ring are independently and optionally substituted by a heteroatom. The heteroatom has the meaning as described in the present invention, and the ring may be completely saturated or contain one or more unsaturated degrees, but is by no means aromatic, and only one attachment point is connected to the rest of the molecule. Hydrogen atoms on one or more rings are independently and optionally substituted by one or more substituents described in the present invention. Some of these embodiments are that the "heterocycle", "heterocyclyl", "heteralicyclic group", or "heterocyclic" group is a single cyclic ring of 3-7 membered ring (1-6 carbon atoms and 1-3 heteroatoms selected from the group consisting of N, O, P, and S, wherein S or P is optionally substituted by one or more oxygen atoms to obtain a group such as SO, SO₂, PO, or PO₂, in which there is only one heteroatom when the ring is a tricyclic ring), or a 7-10 membered bicyclic ring (4-9 carbon atoms and 1-3 heteroatoms selected from the group consisting of N, O, P, and S, wherein S or P is optionally substituted by one or more oxygen atoms to obtain a group such as SO, SO₂, PO, or PO₂).

The heterocyclic groupcan be either carbon group or heteroatom group. " heterocyclyl " also include group formed by the merger of heterocyclic groups with saturated or partially unsaturated rings or heterocycle. Examples of heterocycles include, but not limited to, pyrrolidyl, tetrahydrofuranyl, dihydrofuranyl, tetrahydrothiophenyl, tetrahydropyranyl, dihydropyranyl, tetrahydrothianyl, piperidinyl, morpholinyl, thiomorpholinyl, thioxanyl, thiazolidinyl, oxazolidinyl, piperazinyl, homopiperazinyl, azacyclobutyl, oxacyclobutyl, thiacyclobutyl, piperidinyl, homopiperidinyl, epoxypropyl, azacycloheptyl, oxacycloheptyl, thiacycloheptyl, 4-methoxy-piperidin-1-yl, 1,2,3,6-tetrahydropyridine-1-yl, oxazepinyl, diazepinyl, thiazepinyl, pyrroline-1-yl, 2-pyrrolinyl, 3-pyrrolinyl, dihydroindolyl, 2H-pyranyl, 4H-pyranyl, dioxacyclohexyl, 1,3-dioxamyl, pyrazolinyl, dithianyl, dithiolanyl, dihydrothiophenyl, pyrazolylimidazolinyl, imidazolidinyl, 1,2,3,4-tetrahydroisoquinolinyl, 1,2,6-thiandiazine-1, 1-dioxo-2-yl, 4-hydroxy-1,4-azaphosphane-4-oxide-1-yl, 2-hydroxy-1 -(piperazine-1 -yl)ethylketo-4-yl, 2-hydroxy-1-(5,6-dihydro-1,2,4-triazin-1(4H)-yl)ethylketo-4-yl, 5,6-dihydro-4H-1,2,4-oxadiazine-4-yl, 2-hydroxy-1-(5,6-dihydropyridine-1(2H)-yl)ethylketo-4-yl, 3-azabicyclo[3.1.0]hexyl, 3-azabicyclo[4.1.0]heptyl, azabicyclo[2.2.2]hexyl, 2-methyl-5,6,7,8-tetrahydro-[1,2,4]triazole[1,5-c]pyrimidin-6-yl, 4,5,6,7-tetrahydroisoxazole[4,3-c]pyridin-5-yl, 3H-indolyl, 2-oxo-5-azabicyclo[2.2.1]heptan-5-yl, 2-oxo-5-azabicyclo[2.2.2]octan-5-yl, quinazinyl, and N-pyridylurea. Examples of the heterocyclic group also include 1,1-dioxothiomorpholinyl, and groups that two carbon atoms on the ring are substituted by oxygen atoms such as the pyrimidine diketol. The heterocyclic group may be substituted or unsubstituted, wherein the substituent may be, but not limited to, oxo (=O), hydroxyl, amino, halogen, cyano, heteroaryl, alkoxy, alkylamino, alkyl, alkenyl, alkynyl, heterocyclyl, sulfhydryl, nitro, aryloxy, hydroxyl-substituted alkoxy, hydroxyl-substituted alkyl-C(=O), alkyl-C(=O), alkyl-S(=O), alkyl-S(=O)₂-, hydroxyl-substituted alkyl-S(=O), hydroxyl-substituted alkyl-S(=O)z, carboxylalkoxy, etc.

The term "aryl" can be used alone or as a large part of "aryl alkyl", "aryl alkoxy" or "aryloxy alkyl" to refer to a single cyclic, bicyclic, or tricyclic carbon ring system containing a total of 6-14 membered rings, wherein at least one ring system is aromatic, each ring system contains 3-7 membered rings, and only one attachment point is connected to the rest of the molecule. The term "aryl" can be used interchangeably with the term "aromatic ring", for example, the aromatic ring can include phenyl, naphthyl, and anthryl. The aryl may be substituted or unsubstituted, wherein the substituent group may be, but not limited to, hydroxyl, amino, halogen, cyano, aryl, heteroaryl, alkoxy, alkylamino, alkyl, alkenyl, alkynyl, heterocyclyl, sulfhydryl, nitro, aryloxy, hydroxyl-substituted alkoxy, hydroxyl-substituted alkyl-C(=O), alkyl-C(=O), alkyl-S(=O), alkyl-S(=O)z-, hydroxyl-substituted alkyl-S(=O), hydroxyl-substituted alkyl-S(=O)z, carboxylalkoxy, etc.

The term "heteroaryl" means a single cyclic, bicyclic, or tricyclic ring system containing a total of 5-14 membered rings, wherein at least one ring system is aromatic, and at least one ring system contains one or more heteroatoms, wherein the heteroatom has the meaning described in the present invention, and each ring system contains 3-7-membered rings, and only one attachment point is connected to the rest of the molecule. The term "heteroaryl" can be used interchangeably with the term "aromatic heterocycle" or "heteroaromatic compound". The heteroaryl may be substituted or unsubstituted, wherein the substituent group may be, but not limited to, hydroxyl, amino, halogen, cyano, aryl, heteroaryl, alkoxy, alkylamino, alkyl, alkenyl, alkynyl, cycloalkyl, heterocyclyl, sulfhydryl, nitro, aryloxy, hydroxyl-substituted alkoxy, hydroxyl-substituted alkyl-C(=O)-, alkyl-C(=O)-, alkyl-S(=O)-, alkyl-S(=O)₂-, hydroxyl-substituted alkyl-S(=O)-, hydroxyl-substituted alkyl-S(=O)z-, carboxylalkoxy, etc.

Other embodiments are that the heteroaryl includes, but not limited to, the following single cyclic rings: 2-furanyl, 3-furanyl, *N*-imidazolyl, 2-imidazolyl, 4-imidazolyl, 5-imidazolyl, 3-isooxazolyl, 4-isooxazolyl, 5-isooxazolyl, 2-oxazolyl, 4-oxazolyl, 5-oxazolyl, 4-methylisoxazole-5-yl, *N-*pyrrolelyl, 2-pyrrolelyl, 3-pyrrolelyl, 2-pyridinyl, 3-pyridinyl, 4-pyridinyl, 2-pyrimidinyl, 4-pyrimidinyl, pyrimidin-5-yl, pyridazinyl (e.g., 3-pyridazinyl), 2-thiazolyl, 4-thiazolyl, 5-thiazolyl, tetrazolyl (e.g., 5-tetrazolyl), triazolyl (e.g., 2-triazolyl and 5-triazolyl), 2-thiophenyl, 3-thiophenyl, pyrazolyl (e.g., 2-pyrazolyl), isothiazolyl, 1,2,3-oxadiazolyl, 1,2,5-oxadiazolyl, 1,2,4-oxadiazolyl, 1,2,3-triazolyl, 1,2,3-thiodiazolyl, 1,3,4-thiodiazolyl, 1,2,5-thiodiazolyl, 1,3,4-thiadiazol-2-yl, pyrazinyl, pyrazin-2-yl, 1,3,5-triazinyl; and also include the following bicyclic rings, but not limited to: benzimidazolyl, benzofuranyl, benzothiophenyl, indolyl (e.g., 2-indolyl), purinyl, quinolinyl (e.g., 2-quinolinyl, 3-quinolinyl, 4-quinolinyl), and isoquinolinyl (e.g., 1-isoquinolinyl, 3-isoquinolinyl, or 4-isoquinolinyl), benzo[d]thiazol-2-yl, imidazo[1,5-a] pyridin-6-yl.

The term "heteroatom" means one or more of O, S, N, P, and Si atoms, including N, S, and P in any form of oxidation state; forms of primary, secondary, tertiary amines and quaternary ammonium salts; or the form in which the hydrogen on the nitrogen atom in the heterocyclic ring is substituted, for example, N (e.g., N in 3,4-dihydro-2h-pyrrolyl), NH (e.g., NH in pyrrolidyl) or NR (e.g., NR in N-substituted pyrrolidyl).

The term "heteroalkyl" refers to that one or more heteroatoms can be inserted in the middle of the alkyl chain, wherein the alkyl and heteroatom have the meaning as described in the present invention. Unless otherwise specified, the heteroalkyl group contains 1-10 carbon atoms, in some other embodiments the heteroalkyl group contains 1-8 carbon atoms, in some other embodiments the heteroalkyl group contains 1-6 carbon atoms, and in some other embodiments the heteroalkyl group contains 1-4 carbon atoms, and in some other embodiments the heteroalkyl group contains 1-3 carbon atoms. Such examples include, but not limited to, CH₃OCH₂-, CH₃CH₂OCH₂-, CH₃SCH₂-, CH₃SCH₂CH₂-, (CH₃)₂NCH₂-, (CH₃)₂CH₂OCH₂-, CH₃OCH₂CH₂-, CH₃CH₂OCH₂CH₂-, etc.

The term "halogen" refers to F, Cl, Br or I.

The "halogenated" of the present invention means that the following group is substituted by a halogen, and the number of substitutions may be one or more.

The term "hydroxyl-substituted" of the present invention means that the following group is substituted by a hydroxyl group, and the number of substitutions may be one or more.

When the term "substituted" described in the present invention is used between two groups, the group in front is a substituent, such as "aryl-substituted alkyl" means that there is an aryl substituent on the alkyl, and "alkyloxy carbonyl-substituted alkyl" means that the alkyl has an alkoxy carbonyl substituent.

When multiple groups in the present invention are used in combination, from left to right, the substitution relationship is sequentially, such as "aryl alkyl", which represents alkyl-substituted aryl, and "alkoxy alkoxy", which represents alkoxy-substituted alkoxy.

### Example 1

The present invention provided an eye-drop, which was composed of the following components:

| Component | Weight |
|---|---|
| 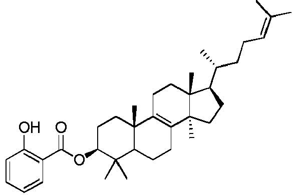 | 20 mg |
| HPMC E5 | 0.12 g |
| Poloxamer P407 | 2.05 g |
| Poloxamer P188 | 0.16 g |
| Water | Added to 10 mL |

The preparation method was as follows:
Under aseptic operation, the above-mentioned components were mixed, and stirred at 2 to 8 °C overnight to obtain a homogeneous mixture. The homogeneous mixture was stored in an eye medicine bottle.

### Example 2

This example provided a method for using the eye-drop provided in Example 1.

The eye-drop provided in Example 1 was used every day, four times per day and 1 to 2 drops each time.

### Effect testing

10 patients with floaters were selected for clinical effect evaluation. Since the date of diagnosis, the patients used the eye-drop provided in Example 1 four times per day and 1 to 2 drops each time, and were revisited after Day 15 and Day 30 of administration. The treatment effects are shown in Table 1 below.

**Table 1**

| Patient No. | Symptoms | After 15 days of administration | After 30 days of administration |
|---|---|---|---|
| 1 | Floaters: moderate vitreous opacity, more than 10 fluttering black dots in front of the eyes, fluttering with the rotation of the eyeball, dust-like floating objects in the anterior vitreous body can be seen under the slit lamp; the observation of the white background such as ceilings and walls was blurred, and the original color cannot be seen clear, affecting vision. | The fluttering black dots were reduced and shallower than before administration, the floating objects in the anterior vitreous body were reduced, the observation of the white background such as ceilings and walls was clearer than before administration, and the contrast sensitivity was improved. | The black dots almost disappeared, the floating objects in the anterior vitreous body basically disappeared, and the visual quality such as contrast sensitivity was improved significantly. |
| 2 | Floaters: mild vitreous opacity, 3-4 fluttering black dots in front of the eyes, fluttering with the rotation of the eyeball, and the observation of the white background such as ceilings and walls was affected. | The fluttering black dots were shallower than before administration, and the observation of the white background such as ceilings and walls was clearer than before administration. | The fluttering black dots disappeared and the visual quality such as contrast sensitivity was improved significantly. |
| 3 | Floaters: moderate vitreous opacity, 5-6 fluttering black dots in front of the eyes, dust-like floating objects in the anterior vitreous body can be seen under the slit lamp, and the observation of the white background such as ceilings and walls was affected. | The fluttering black dots were shallower than before administration, the floating objects in the anterior vitreous body were reduced, the observation of the white background such as ceilings and walls was clearer than before administration, and the contrast sensitivity was improved. | The black dots almost disappeared, the floating objects in the anterior vitreous body basically disappeared, and the visual quality such as contrast sensitivity was improved significantly. |
| 4 | Floaters: mild vitreous opacity, 4-5 fluttering black dots in front of the eyes, and the observation of the white background such as ceilings and walls was affected or interfered. | The fluttering black dots were shallower than before administration, the observation of the white background such as ceilings and walls was clearer than before administration, and the contrast sensitivity was improved. | The black dots almost disappeared, and the visual quality such as contrast sensitivity was improved significantly. |
| 5 | Floaters: mild vitreous opacity, 3-4 fluttering black dots in front of the eyes, and the observation of the white background such as ceilings and walls was affected or interfered. | The fluttering black dots were shallower than before administration, the observation of the white background such as ceilings and walls was clearer than beforeadministration, and the contrast sensitivity was improved. | The black dots almost disappeared, and the visual quality such as contrast sensitivity was improved significantly. |
| 6 | Floaters: moderate vitreous opacity, 7-8 fluttering black dots in front of the eyes, 1-2 bands of transparent opacity, the observation of the white background such as ceilings and walls was affected greatly, and there was visual disturbance. | The fluttering black dots were reduced to 3-4, at the same time, the transparent bands were obviously shallower, the observation of the white background such as ceilings and walls was clearer than before administration, and the disturbance was reduced. | The black dots almost disappeared, the bands almost disappeared, and the visual quality such as contrast sensitivity was improved significantly. |
| 7 | Floaters: mild vitreous opacity, 3-4 fluttering black dots in front of the eyes, the observation of the white background such as ceilings and walls was affected, and there was visual disturbance. | The fluttering black dots were shallower than before administration, the observation of the white background such as ceilings and walls was clearer than before administration, and the contrast sensitivity was improved. | The black dots disappeared, the vision was clearer, and the visual quality such as contrast sensitivity was improved significantly. |
| 8 | Floaters: moderate vitreous opacity, 6-7 fluttering black dots in front of the eyes, 1 band fluttering, the observation of the white background such as ceilings and walls was affected, and there was visual disturbance. | The fluttering black dots and band were shallower than before administration, the observation of the white background such as ceilings and walls was clearer than before administration, and the contrast sensitivity was improved. | Both the black dots and the band disappeared, and the observation of the white background such as ceilings and walls was unaffected, and the visual quality such as contrast sensitivity was improved significantly. |
| 9 | Floaters: mild vitreous opacity, 4-5 fluttering black dots in front of the eyes, and the observation of the white background such as ceilings and walls was affected. | The fluttering black dots were shallower than before administration, the observation of the white background such as ceilings and walls was clearer than before administration, and the contrast sensitivity was improved. | The black dots almost disappeared, and the observation of the white background such as ceilings and walls was unaffected, and the visual quality such as contrast sensitivity was improved significantly. |
| 10 | Floaters: mild vitreous opacity, 3-4 fluttering black dots in front of the eyes, and the observation of the white background such as ceilings and walls was affected. | The fluttering black dots were shallower than before administration, the observation of the white background such as ceilings and walls was clearer than before administration, and the contrast sensitivity was improved. | The black dots almost disappeared, and the observation of the white background such as ceilings and walls was unaffected, and the visual quality such as contrast sensitivity was improved significantly. |

It can be seen from Table 1, in terms of clinical use effect, the black dots or bands in front of eyes were obviously shallower and the contrast sensitivity was improved, and the visual disturbance when observing white background such as ceilings and walls was reduced after about 15 days of administration; after about 30 days of administration, floaters can be basically cured, fluttering black dots or bands almost disappeared, there was no visual disturbance when observing white background such as ceilings and walls, the visual quality such as contrast sensitivity was improved significantly, and there were no other side effects.

Although the present invention has been described in detail with general description, specific embodiments and tests, on the basis of the present invention, some modifications or improvements can be made, which are obvious to those skilled in the art. Therefore, these modifications or improvements made on the basis of not deviating from the spirit of the present invention belong to the scope of protection claimed by the present invention.

## Claims

1. Use of a steroidal compound in the preparation of a medicament for preventing and/or treating floaters, wherein the steroidal compound has a structure shown in formula I, or is a stereoisomer, tautomer, nitrogen oxide, solvate, metabolite, pharmacologically acceptable salt or prodrug of the compound of the structure shown in formula I; 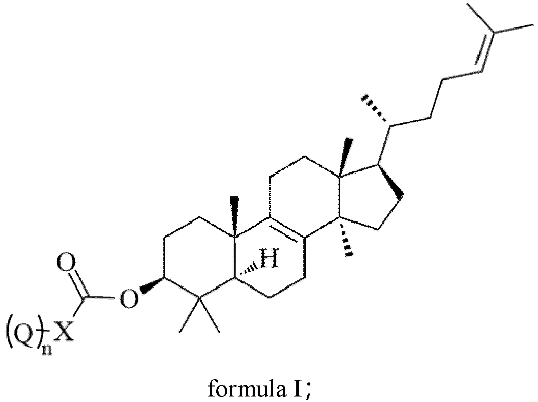
wherein, X is selected from the group consisting of aryl, heteroaryl, cycloalkyl, heterocycloalkyl, heteroalkyl, substituted or unsubstituted alkyl, and the substituted group is selected from the group consisting of hydroxyl, sulfhydryl, amino, aryl, heteroaryl, carboxyl, R¹R²NC(=O)-, and R¹R²NC(=NH)-NR³-;
each Q is independently selected from the group consisting of -H, -D, halogen, hydroxyl, amino, cyano, nitro, carboxyl, alkyl carbonyl, alkyl, haloalkyl, cycloalkyl, heterocyclyl, aryl, and heteroaryl;
n is 0, 1, 2, or 3;
R¹, R², and R³ are independently selected from the group consisting of -H, -D, and alkyl.

2. The use according to claim 1, wherein X is selected from the group consisting of C₆₋₁₀ aryl, C₂₋₉ heteroaryl, C₃₋₈ cycloalkyl, C₂₋₁₀ heterocycloalkyl, C₁₋₆ heteroalkyl, substituted or unsubstituted C₁₋₆ alkyl, and if the C₁₋₆ alkyl has a substituent group, the substituent group is selected from the group consisting of hydroxyl, sulfhydryl, amino, C₆₋₁₀ aryl, C₂₋₉ heteroaryl, carboxyl, R¹R²NC(=O)-, and R¹R²NC(=NH)-NR³-;
R¹, R², and R³ are independently selected from the group consisting of -H, -D, and C₁₋₆ alkyl.

3. The use according to claim 2, wherein X is selected from the group consisting of methyl, ethyl, propyl, isopropyl, n-butyl, isobutyl, tert-butyl, hydroxymethyl, hydroxyethyl, mercaptomethyl, mercaptoethyl, aminomethyl, aminoethyl, aminopropyl, phenyl methyl, phenyl ethyl, imidazolyl methyl, carboxymethyl, carboxyethyl, methylthiomethyl, methylthioethyl, phenyl, naphthyl, cyclopropyl, cyclobutyl, cyclopentyl, cyclohexyl, tetrahydrofuranyl, dihydrofuranyl, tetrahydrothiophenyl, dihydrothiophenyl, 1,3-dioxycyclopentyl, dithiocyclopentyl, tetrahydropyranyl, dihydropyranyl, 2H-pyranyl, 4H-pyranyl, tetrahydrothianyl, piperidinyl, morpholinyl, thiomorpholinyl, piperazinyl, dioxanyl, dithianyl, thioxanyl, homopiperazinyl, homopiperidinyl, oxacycloheptyl, thiacycloheptyl, oxazanyl, diazanyl, thiazanyl, indolinyl, 1,2,3,4-tetrahydroquinolinyl, 1,2,3,4-tetrahydroisoquinolinyl, furanyl, imidazolyl, 3-isooxazolyl, isooxazolyl, oxazolyl, pyrrolyl, pyridinyl, pyrimidinyl, pyridazinyl, thiazolyl, tetrazolyl, triazolyl, 2-thiophenyl, 3-thiophenyl, pyrazolyl, isothiazolyl, 1,2,3-oxadiazolyl, 1,2,5-oxadiazolyl, 1,2,4-oxadiazolyl, 1,2,3-triazolyl, 1,2,3-thiodiazolyl, 1,3,4-thiodiazolyl, 1,2,5-thiodiazolyl, pyrazinyl, 1,3,5-triazinyl, benzimidazolyl, benzofuranyl, benzothiophenyl, indolyl, purinyl, quinolinyl, isoquinolinyl, imidazolo[1,2-a]pyridinyl, pyrazolo[1,5-a]pyridinyl, pyrazolo[1,5-a]pyrimidinyl, imidazolo[1,2-b]pyridazinyl, [1,2,4]triazolo[4,3-b]pyridazinyl, [1,2,4]triazolo[1,5-a]pyrimidinyl, [1,2,4]triazolo[1,5-a]pyridinyl, 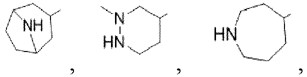 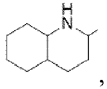 C₁₋₃ alkyl with H₂NC(=O)-substituent or C₁₋₃ alkyl with H₂NC(=NH)-NH-substituent.

4. The use according to claim 1, wherein each Q is independently selected from the group consisting of -H, -D, halogen, hydroxyl, amino, cyano, nitro, carboxyl, C₁₋₆ alkyl carbonyl, C₁₋₆ alkyl, C₁₋₆ haloalkyl, C₃₋₈ cycloalkyl, C₂₋₉ heterocyclyl, C₆₋₁₀ aryl, and C₂₋₉ heteroaryl.

5. The use according to claim 4, wherein each Q is independently selected from the group consisting of -H, -D, -F, -Cl, -Br, hydroxyl, amino, cyano, carboxyl, formyl, acetyl, methyl, ethyl, propyl, isopropyl, n-butyl, isobutyl, tert-butyl, trifluoromethyl, difluoromethyl, phenyl, naphthyl, cyclopropyl, cyclobutyl, cyclopentyl, cyclohexyl, tetrahydrofuranyl, dihydrofuranyl, tetrahydrothiophenyl, dihydrothiophenyl, 1,3-dioxycyclopentyl, dithiocyclopentyl, tetrahydropyranyl, dihydropyranyl, 2H-pyranyl, 4H-pyranyl, tetrahydrothianyl, piperidinyl, morpholinyl, thiomorpholinyl, piperazinyl, dioxanyl, dithianyl, thioxanyl, homopiperazinyl, homopiperidinyl, oxacycloheptyl, thiacycloheptyl, oxazanyl, diazanyl, thiazanyl, indolinyl, 1,2,3,4-tetrahydroquinolinyl, 1,2,3,4-tetrahydroisoquinolinyl, furanyl, imidazolyl, 3-isooxazolyl, isooxazolyl, oxazolyl, pyrrolyl, pyridinyl, pyrimidinyl, pyridazinyl, thiazolyl, tetrazolyl, triazolyl, 2-thiophenyl, 3-thiophenyl, pyrazolyl, isothiazolyl, 1,2,3-oxadiazolyl, 1,2,5-oxadiazolyl, 1,2,4-oxadiazolyl, 1,2,3-triazolyl, 1,2,3-thiodiazolyl, 1,3,4-thiodiazolyl, 1,2,5-thiodiazolyl, pyrazinyl, 1,3,5-triazinyl, benzimidazolyl, benzofuranyl, benzothiophenyl, indolyl, purinyl, quinolinyl, isoquinolinyl, imidazolo[1,2-a]pyridinyl, pyrazolo[1,5-a]pyridinyl, pyrazolo[1,5-a]pyrimidinyl, imidazolo[1,2-b]pyridazinyl, [1,2,4]triazolo[4,3-b]pyridazinyl, [1,2,4]triazolo[1,5-a]pyrimidinyl, and [1,2,4]triazolo[1,5-a]pyridinyl.

6. The use according to any one of claims 1 to 5, wherein the compound of the structure shown in formula I is selected from the group consisting of any one of the following compounds:

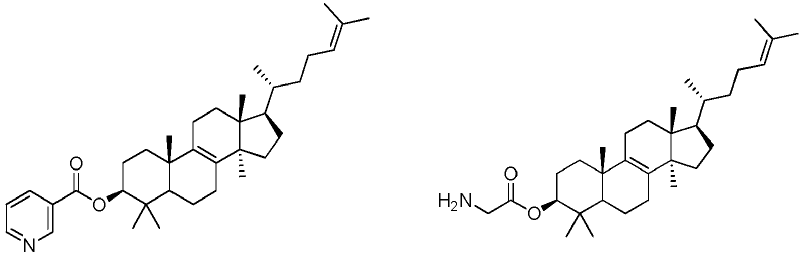

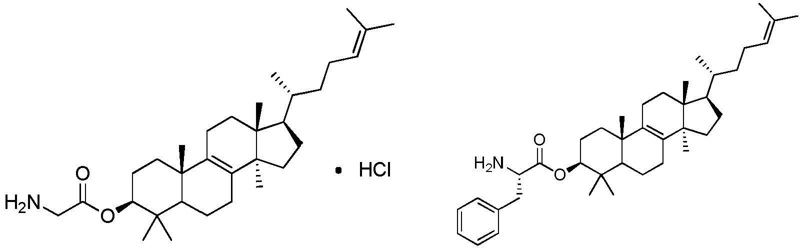

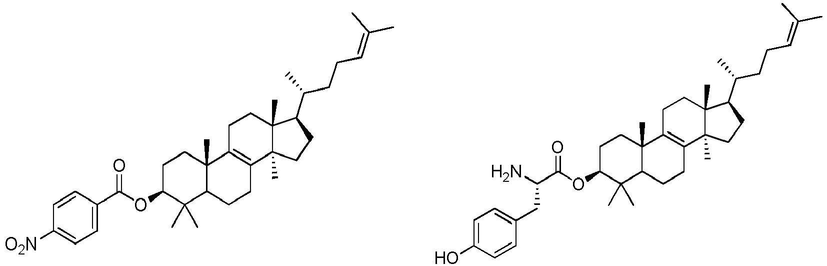

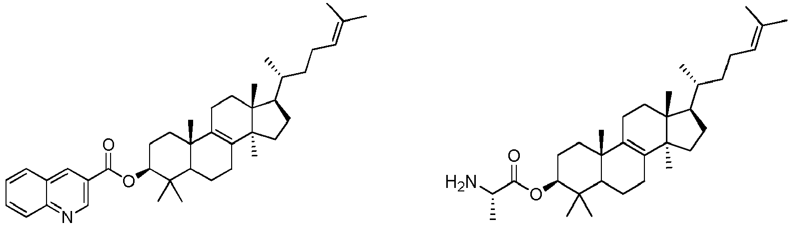

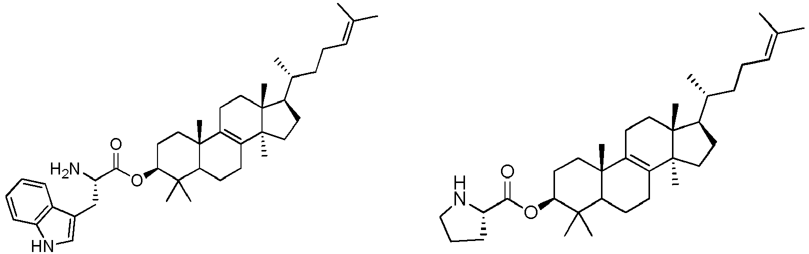

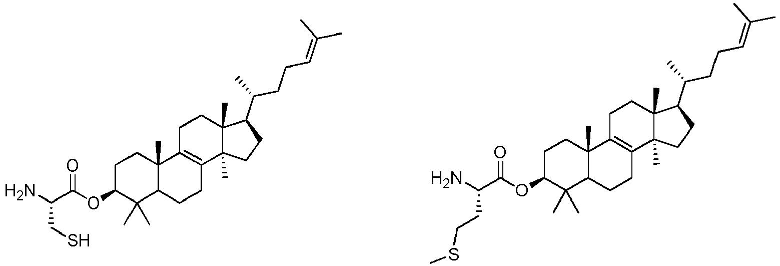

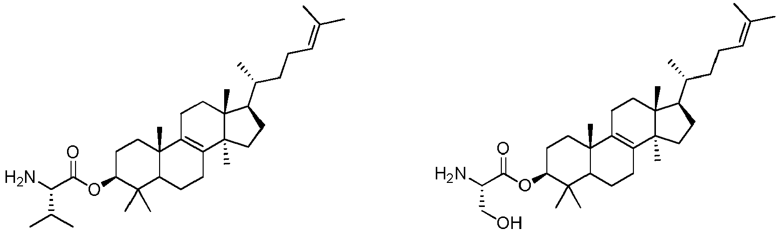

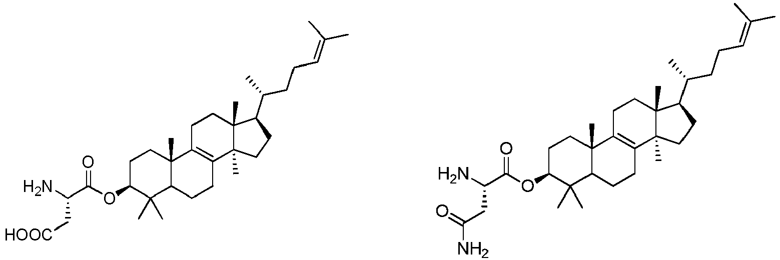

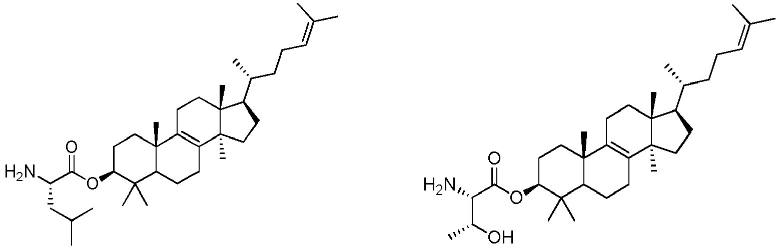

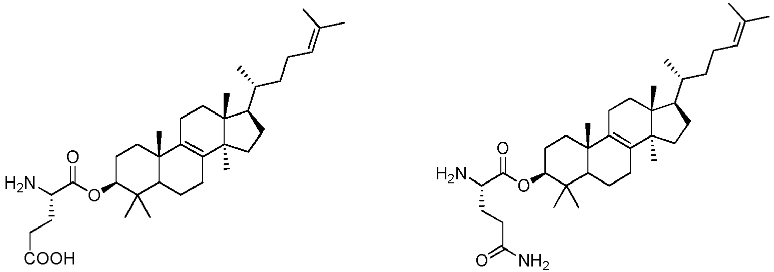

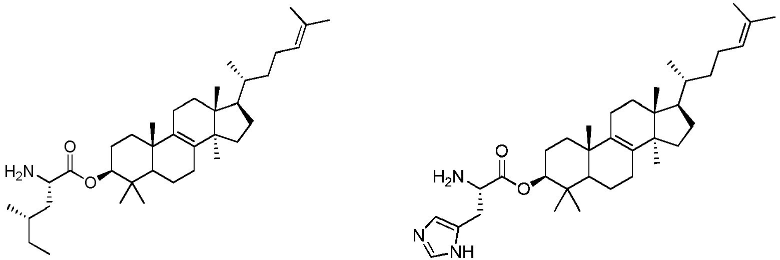

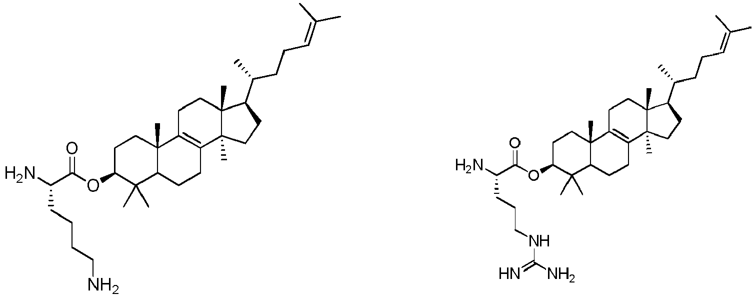

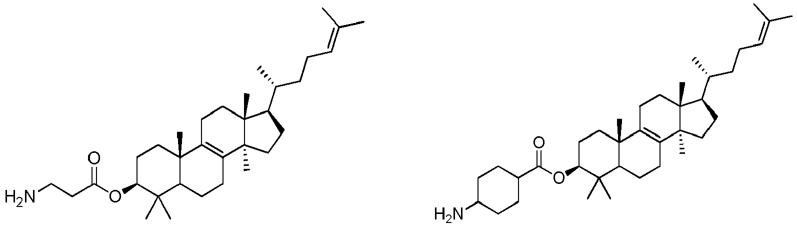

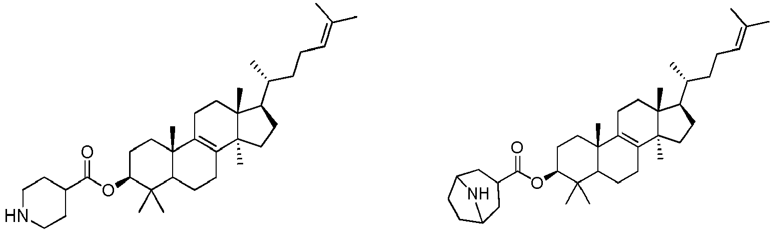

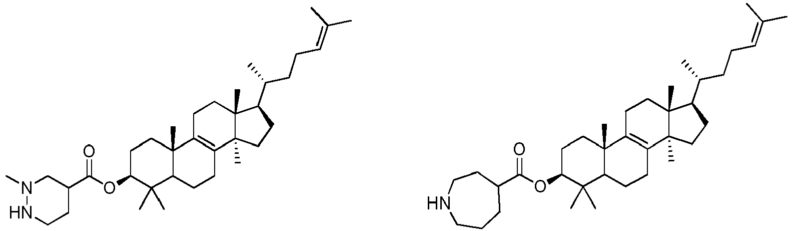

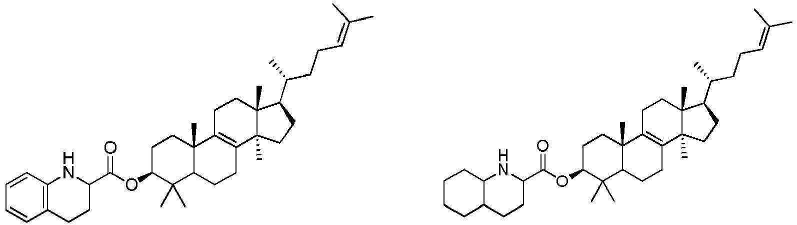

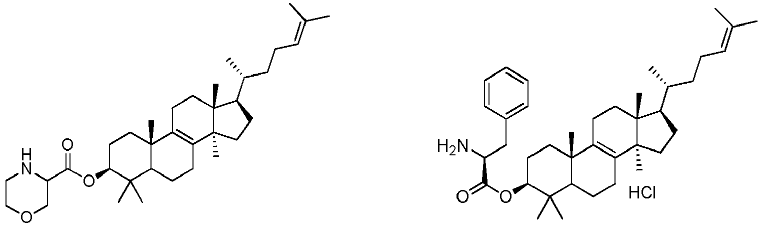

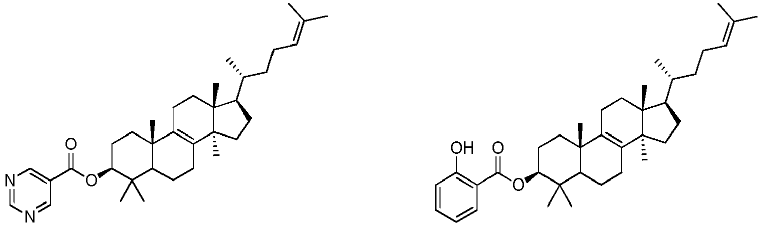

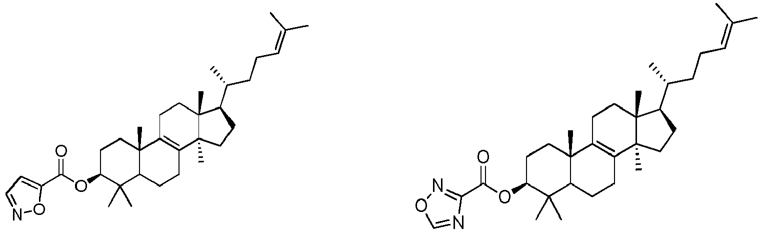

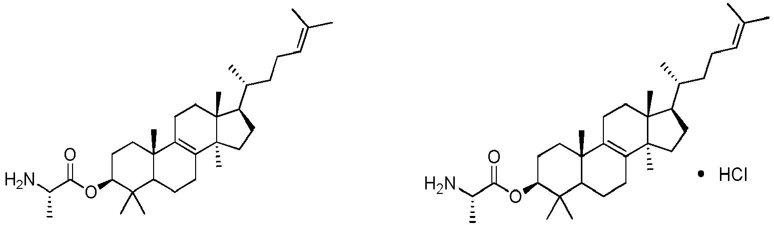

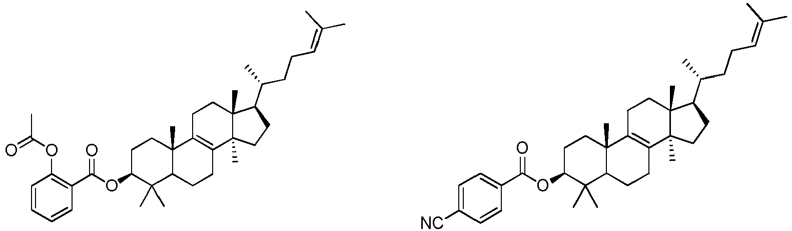

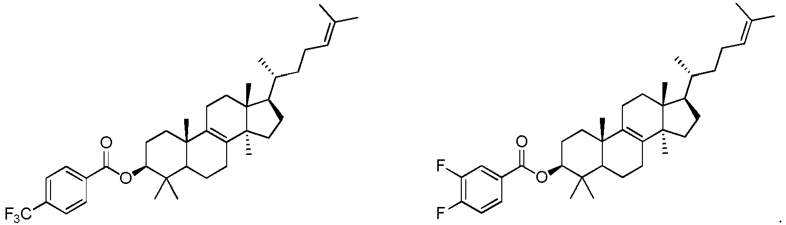

7. Use of a composition in the preparation of a medicament for preventing and/or treating eye floaters, wherein the composition comprises the steroidal compound according to any one of claims 1 to 6, and one or more of the pharmaceutically acceptable carriers, excipients, diluents, adjuvants or vehicles.
